(19) 

Europäisches Patentamt
European Patent Office
Office européen des brevets

(11) **EP 4 741 495 A1**

(12) **EUROPEAN PATENT APPLICATION**
published in accordance with Art. 153(4) EPC

(43) Date of publication:
**13.05.2026   Bulletin 2026/20**

(21) Application number: **24843156.1**

(22) Date of filing: **17.07.2024**

(51) International Patent Classification (IPC):
*C12N 5/071* (2010.01)    *C12M 3/00* (2006.01)

(52) Cooperative Patent Classification (CPC):
**C12M 3/00; C12N 5/06**

(86) International application number:
**PCT/JP2024/025676**

(87) International publication number:
**WO 2025/018367 (23.01.2025 Gazette 2025/04)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **20.07.2023   JP 2023118584**

(71) Applicant: **Canon Kabushiki Kaisha
Tokyo 146-8501 (JP)**

(72) Inventors:
• **TAKEMURA, Kenjiro
Yokohama-shi, Kanagawa 223-8522 (JP)**

• **KURASHINA, Yuta
Yokohama-shi, Kanagawa 223-8522 (JP)**
• **IMASHIRO, Chikahiro
Yokohama-shi, Kanagawa 223-8522 (JP)**
• **TSUBAKI, Keiichiro
Tokyo 146-8501 (JP)**
• **FURUI, Takaaki
Tokyo 146-8501 (JP)**
• **OGUCHI, Yuichiro
Tokyo 146-8501 (JP)**
• **NOGUCHI, Kazunori
Tokyo 146-8501 (JP)**

(74) Representative: **WESER & Kollegen
Patentanwälte PartmbB
Radeckestraße 43
81245 München (DE)**

(54) **CELL DETACHMENT METHOD AND CELL DETACHMENT APPARATUS**

(57)   To provide a cell detachment method and cell detachment device capable of designing a vibration in accordance with a target cell by mechanically matching a culture substrate with a vibrating body, and detaching an adherent cell at a high detachment rate. A cell detachment method for detaching cells attached to a culture surface of a culture substrate from the culture surface, wherein the cell detachment method detaches the cells from the culture surface by vibrating a vibrating body in a state where an acoustic transmission medium is provided between the culture substrate and the vibrating body, wherein the acoustic transmission medium comprises a first acoustic transmission medium and a second acoustic transmission medium, wherein the first acoustic transmission medium is a solid having elasticity at room temperature, and the second acoustic transmission medium is encapsulated within the first acoustic transmission medium and is liquid at room temperature or has a tan$\delta$ greater than that of the first acoustic transmission medium when vibration of frequency 0.1 Hz is applied to the second acoustic transmission medium at room temperature.

FIG. 1A

## Description

[Technical Field]

The present disclosure relates to a cell detachment method and a cell detachment device.

[Background Art]

In recent years, cell culture for culturing cells used in regenerative medicine and cells used for manufacturing biopharmaceuticals and the like has been actively conducted. Cell culture is broadly classified into adherent culture and suspension culture, of which adherent culture is suitable for various cell types and is widely used. However, when the cells are collected, it is necessary to perform enzyme treatment in order to detach the cells, and depending on the treatment method, the cells may be damaged. Therefore, cell detachment by enzyme treatment is also a cause of dispersion in cell quality. Therefore, detachment conditions for collecting cells at a high detachment rate without damaging the cells have been studied.

In the case of cell detachment techniques using ultrasonic waves or acoustic radiation pressure, water is generally used as the acoustic transmission medium, and in this case, water tends to be particularly avoided in medical practice because of the strong concern about the propagation of bacteria.

In Patent Literature 1, it is proposed that a patterning device for arranging cells in specific positions via a medium for transmitting acoustic radiation pressure to a culture substrate. Silicone rubber is described as an example of the acoustic transmission medium, but when the acoustic radiation pressure is transmitted to the culture substrate using this material, the cell detachment performance is not sufficient compared with water, and further improvement of the cell detachment performance and reduction of dispersion are required. Also in Patent Literature 2, a medical ultrasonic transmission device composed of a closed body is proposed. Although used for acoustic coupling between the ultrasonic probe and the target site, there is no description of using this ultrasonic transmission method to transmit ultrasonic vibrations and detach cells.

[Citation List]

[Patent Literature]

PTL 1: Japanese Patent Laid-Open No. 2018-42534
PTL 2: Japanese Patent No. 6325363

[Summary of Invention]

[Technical Problem]

The present disclosure has been made to solve the above problems, and provides a cell detachment method that enables vibration design in accordance with target cells and detachment of adherent cells at a high detachment rate.

[Solution to Problem]

The present disclosure provides a cell detachment method for detaching cells attached to a culture surface of a culture substrate from the culture surface,

wherein the cell detachment method detaches the cells from the culture surface by vibrating a vibrating body in a state where an acoustic transmission medium is provided between the culture substrate and the vibrating body,
wherein the acoustic transmission medium comprises a first acoustic transmission medium and a second acoustic transmission medium,
wherein the first acoustic transmission medium is a solid having elasticity at room temperature,
and the second acoustic transmission medium is encapsulated within the first acoustic transmission medium and is liquid at room temperature or has a $\tan\delta$ greater than that of the first acoustic transmission medium when vibration of frequency 0.1 Hz is applied to the second acoustic transmission medium at room temperature.

Also, the present disclosure provides a cell detachment device for detaching cells attached to a culture surface of a culture substrate by transmitting vibrations generated by a vibrating body to the cells, comprising

a vibrating body,
an acoustic transmission medium, and
a culture substrate installation portion in this order,
and configured so that vibrations of the vibrating body are transmitted to the culture substrate via the acoustic transmission medium,
wherein the acoustic transmission medium comprises a first acoustic transmission medium and a second acoustic transmission medium,
wherein the first acoustic transmission medium is a solid having elasticity at room temperature,
and the second acoustic transmission medium is encapsulated within the first acoustic transmission medium and is liquid at room temperature or has a $\tan\delta$ greater than that of the first acoustic transmission medium when vibration of frequency 0.1 Hz is applied to the second acoustic transmission medium at room temperature.

[Advantageous Effects of Invention]

According to the present disclosure, it is possible to provide a cell detachment method that enables vibration design in accordance with target cells and detachment of adherent cells at a high detachment rate.

[Brief Description of Drawings]

[Fig. 1A]

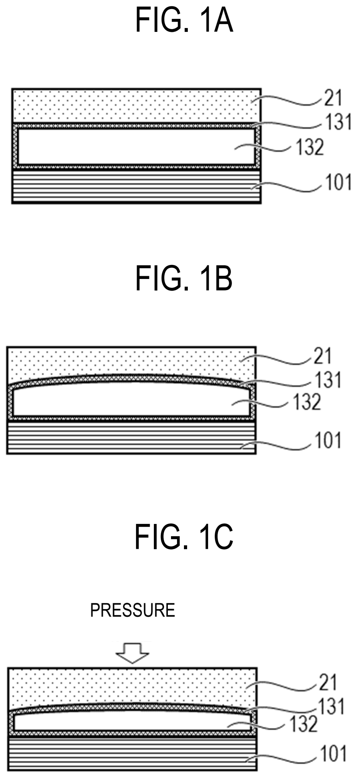

Fig. 1A is a schematic cross-sectional view showing an example of a configuration of a first embodiment of a cell detachment device according to the present disclosure.
[Fig. 1B]
Fig. 1B is a schematic cross-sectional view showing an example of the configuration of the first embodiment of the cell detachment device according to the present disclosure.
[Fig. 1C]
Fig. 1C is a schematic cross-sectional view showing an example of the configuration of the first embodiment of the cell detachment device according to the present disclosure.
[Fig. 2A]

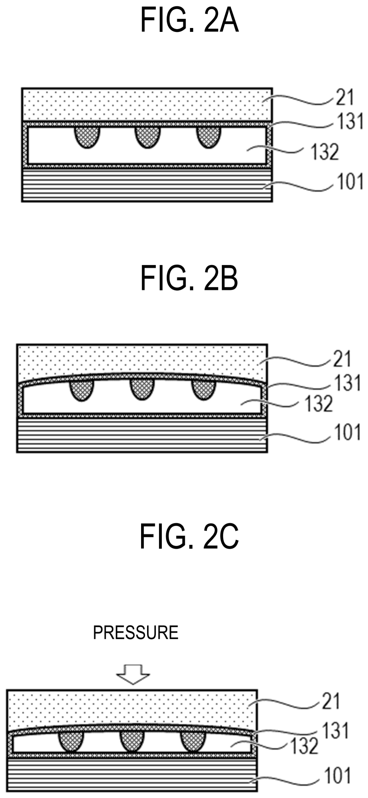

Fig. 2A is a schematic cross-sectional view showing an example of a configuration of a second embodiment of the cell detachment device according to the present disclosure.
[Fig. 2B]
Fig. 2B is a schematic cross-sectional view showing an example of the configuration of the second embodiment of the cell detachment device according to the present disclosure.
[Fig. 2C]
Fig. 2C is a schematic cross-sectional view showing an example of the configuration of the second embodiment of the cell detachment device according to the present disclosure.
[Fig. 3A]

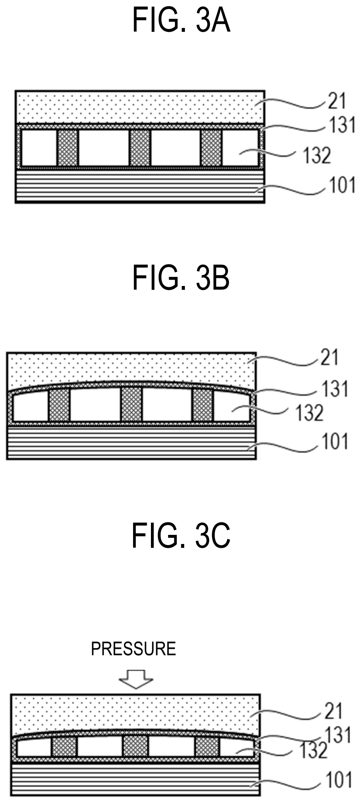

Fig. 3A is a schematic cross-sectional view showing an example of a configuration of a third embodiment of the cell detachment device according to the present disclosure.
[Fig. 3B]
Fig. 3B is a schematic cross-sectional view showing an example of the configuration of the third embodiment of the cell detachment device according to the present disclosure.
[Fig. 3C]
Fig. 3C is a schematic cross-sectional view showing an example of the configuration of the third embodiment of the cell detachment device according to the present disclosure.
[Fig. 4A]

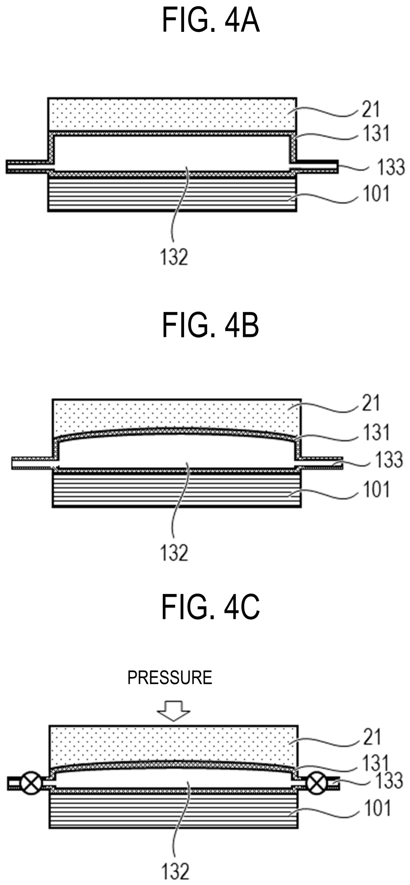

Fig. 4A is a schematic cross-sectional view showing an example of a configuration of a fourth embodiment of the cell detachment device according to the present disclosure.
[Fig. 4B]
Fig. 4B is a schematic cross-sectional view showing an example of the configuration of the fourth embodiment of the cell detachment device according to the present disclosure.
[Fig. 4C]
Fig. 4C is a schematic cross-sectional view showing an example of the configuration of the fourth embodiment of the cell detachment device according to the present disclosure.

[Fig. 5]

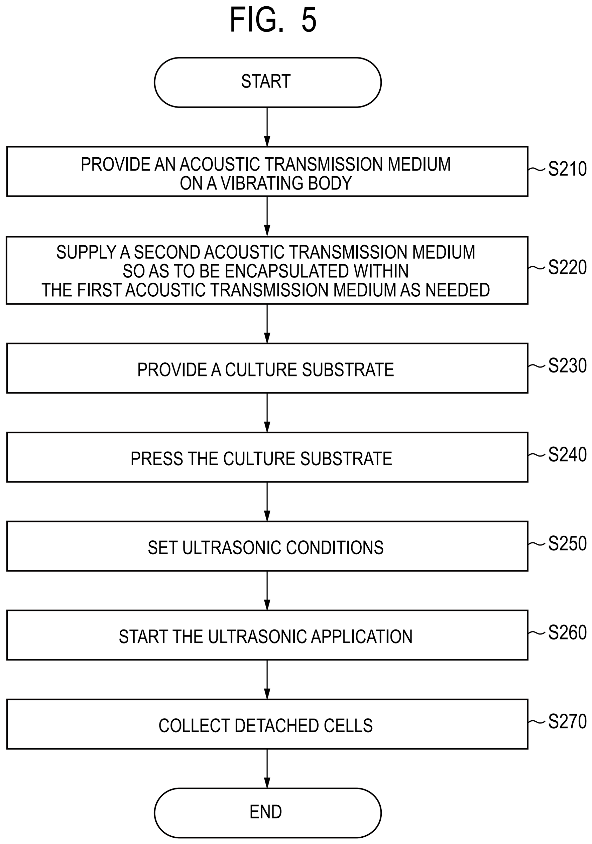

Fig. 5 shows a specific example of steps of a cell detachment method according to the present disclosure.

[Fig. 6]

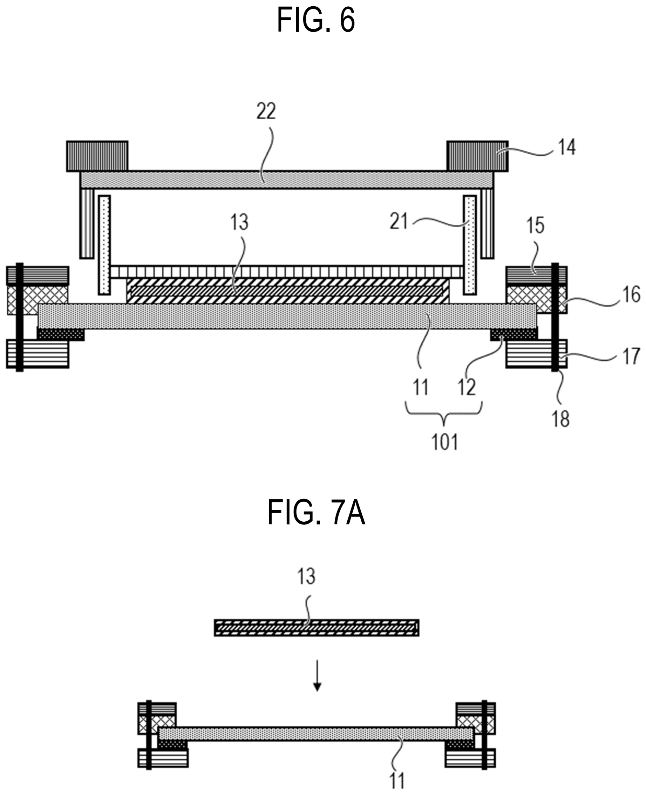

Fig. 6 shows an example of a device according to the present disclosure.

[Fig. 7A]

Fig. 7A illustrates an example of an arrangement step of the method according to the present disclosure.

[Fig. 7B]

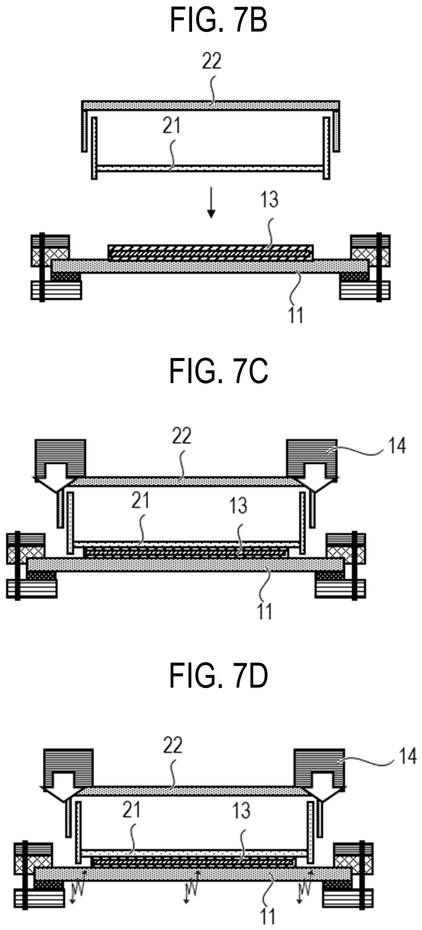

Fig. 7B illustrates an example of the arrangement step of the method according to the present disclosure.

[Fig. 7C]

Fig. 7C illustrates an example of a pressing step of the method according to the present disclosure.

[Fig. 7D]

Fig. 7D illustrates an example of a detachment step of the method according to the present disclosure.

[Fig. 8]

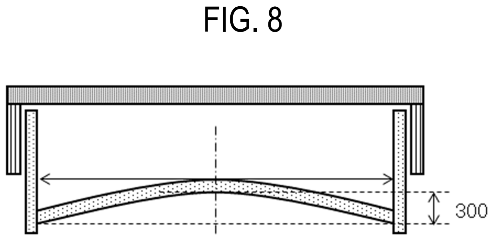

Fig. 8 is a schematic view of an example of a culture substrate according to the present disclosure.

[Fig. 9A]

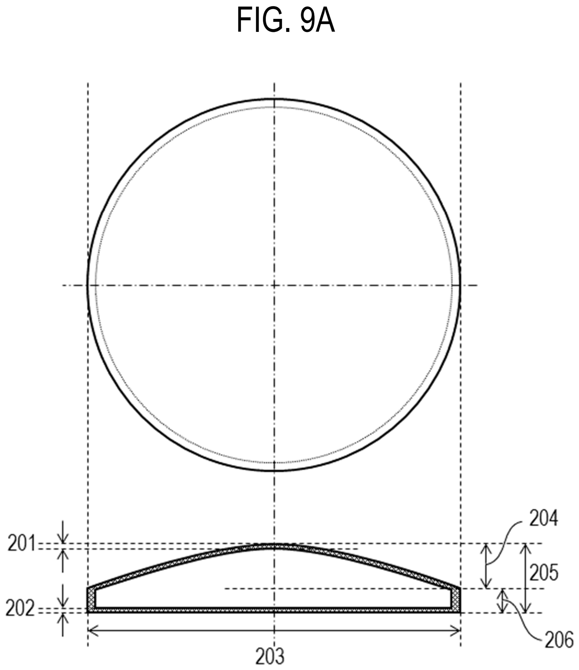

Fig. 9A is a cross-sectional view and a top view illustrating an example of an acoustic transmission medium according to the present disclosure.

[Fig. 9B]

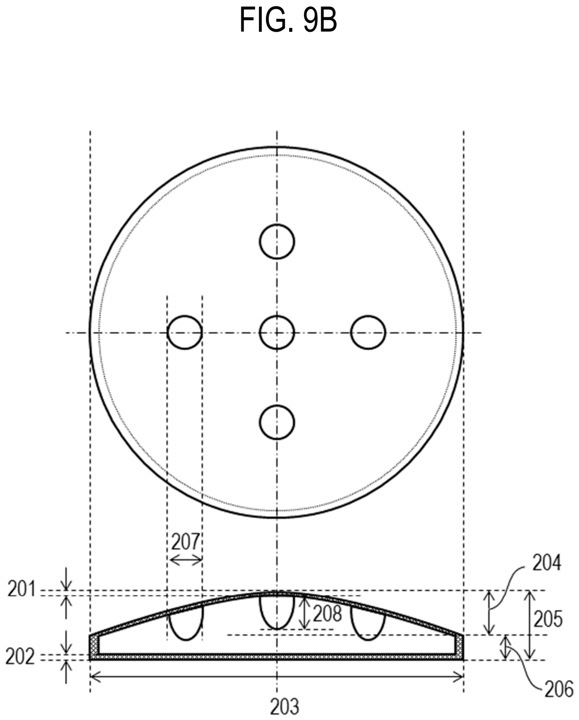

Fig. 9B is a cross-sectional view and a top view illustrating an example of the acoustic transmission medium according to the present disclosure.

[Fig. 9C]

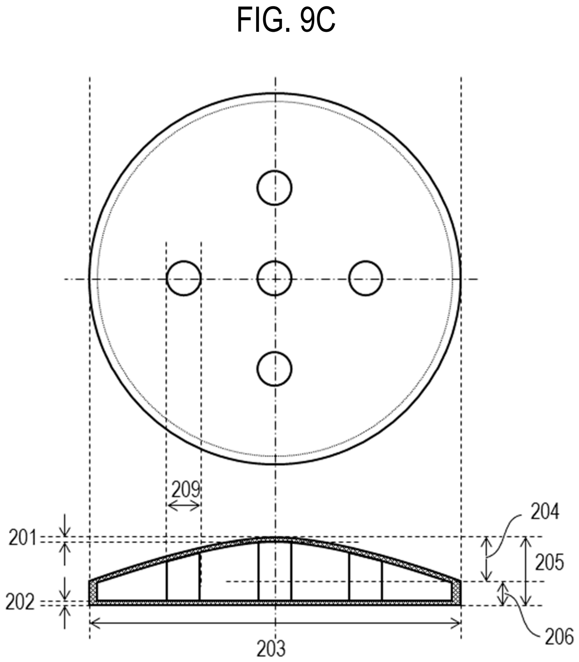

Fig. 9C is a cross-sectional view and a top view illustrating an example of the acoustic transmission medium according to the present disclosure.

[Fig. 9D]

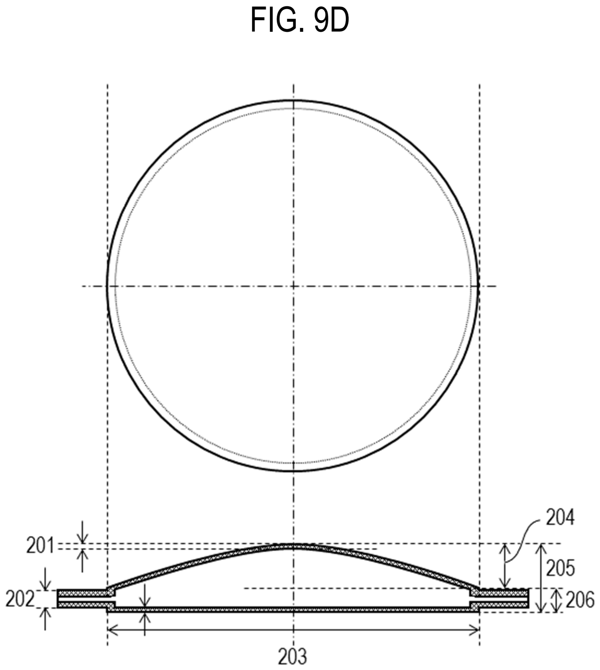

Fig. 9D is a cross-sectional view and a top view illustrating an example of the acoustic transmission medium according to the present disclosure.

[Fig. 10]

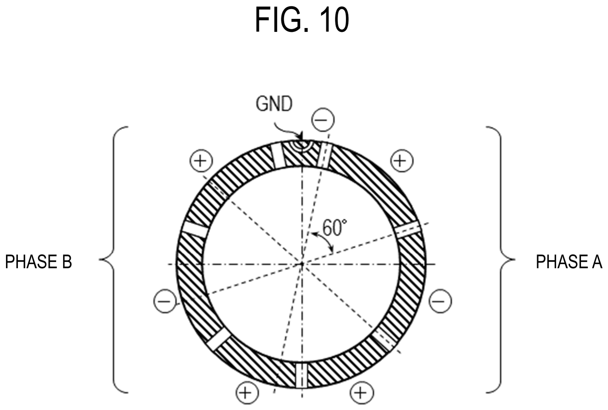

Fig. 10 is a schematic view for explaining an example of a piezoelectric body according to the present disclosure.

[Description of Embodiments]

The cell detachment method of the present disclosure will be described below with reference to examples, but the present disclosure is not limited to the following examples.

(Basic configuration)

The cell detachment method according to the present disclosure is a cell detachment method for detaching cells attached to a culture surface of a culture substrate from the culture surface,

wherein the cell detachment method detaches the cells from the culture surface by vibrating a vibrating body in a state where an acoustic transmission medium is provided between the culture substrate and the vibrating body,
wherein the acoustic transmission medium comprises a first acoustic transmission medium and a second acoustic transmission medium,
wherein the first acoustic transmission medium is a solid having elasticity at room temperature,
and the second acoustic transmission medium is encapsulated within the first acoustic transmission medium and is liquid at room temperature or has a $\tan\delta$ greater than that of the first acoustic transmission medium when vibration of frequency 0.1 Hz is applied to the second acoustic transmission medium at room temperature.

Conceptual diagrams of the cell detachment method of the present disclosure are shown in Figs. 7A to 7D.

Fig. 7A shows a step of arranging the acoustic transmission medium 13. Note that the acoustic transmission medium 13 may be arranged in advance, and this step is not an essential step for the cell detachment method of this disclosure.

In Fig. 7A, the acoustic transmission medium 13 is arranged on the vibrating body 101. In Fig. 7B, the culture substrate 21 is arranged on the acoustic transmission medium 13. Fig. 7C shows a pressing step, in which pressure is applied from above the culture substrate 21. The pressing step is not essential for the detachment method of this disclosure. Fig. 7D shows a detachment step. In Fig. 7D, cells are detached by applying ultrasonic vibration by driving the vibrating body 101 while applying pressure from above the culture substrate 21. The pressing step makes it easy for the first acoustic transmission medium to be aligned with the culture substrate and the vibrating body, and also in the detachment step, vibration can be transmitted in a state where the first acoustic transmission medium is aligned with the culture substrate and the vibrating body. Here, when the first acoustic transmission medium and the culture substrate or the vibrating body are aligned, it means that the vibration of the vibrating body is in sufficient contact with the culture substrate via the first acoustic transmission medium and the second acoustic transmission medium so as to sufficiently transmit the vibration of the vibrating body to the culture substrate, but an extremely minute gap or an extremely minute amount of material may be interposed between the first acoustic transmission medium and the culture substrate or the vibrating body to the extent that the problem of the present disclosure can be solved. For example, even if a few molecules of water are interposed between the first acoustic transmission medium and the culture substrate, it can be considered that the first acoustic transmission medium and the culture substrate are in contact.

In the cell detachment method of the present disclosure, when the vibration of the vibrating body is transmitted to the culture substrate, an acoustic transmission medium is provided between them. In terms of acoustic transmission medium, a first acoustic transmission medium which has elasticity and is solid at room temperature, encapsulates a second acoustic transmission medium which is liquid at room temperature or has a $\tan\delta$ greater than that of the first acoustic transmission medium ($\tan\delta$ when vibration of frequency 0.1 Hz is applied to the first acoustic transmission medium at room temperature) when vibration of frequency 0.1 Hz is applied to the second acoustic transmission medium at room temperature. The vibrating body as a vibration generating part is provided with a piezoelectric body and a vibration plate, and its vibration mechanism is to generate bending vibration in which vibration is amplified by a combination of hardness between the piezoelectric body which expands and contracts by itself and the bonded vibration plate. In this disclosure, resonance due to the structural factor of the vibrating body is utilized, and large vibration can be obtained at the resonance frequency.

(Acoustic transmission medium)

In the cell detachment method of the present disclosure, an acoustic transmission medium is used to match the vibrating body which generates vibration with the culture substrate to which the vibration is transmitted. In addition to physical matching such as acoustic impedance, mechanical matching such as adhesion is also important for matching. It is desirable that a configuration capable of solving the problem of the present disclosure can be constructed by achieving mechanical matching and physical matching between the acoustic transmission medium and the culture substrate and the vibrating body. In this embodiment, the acoustic transmission medium can also be referred to as an acoustic transmission material.

The physical matching between the vibrating body and the culture substrate can be defined by a physical quantity called "acoustic impedance Z."

Specifically, the acoustic impedance Z of the acoustic transmission medium is defined by Z = (density of the acoustic transmission medium) $\times$ (speed of sound in the acoustic transmission medium).

Furthermore, when a sound wave reaches the boundary between two materials, the transmittance of the sound wave is determined by

$$\text{transmittance} = (4 \times Z1 \times Z2)/(Z1+Z2)^{\wedge}2.$$

Z1 and Z2 are the acoustic impedance of the first and second materials transmitting the sound wave.

For example, the transmittance in water and air and the transmittance in silicone rubber and air are, respectively,

$$\text{water acoustic impedance } Z1 = 1.5 \times 10^{\wedge}6 \text{ [kg/m}^{\wedge}2/\text{s]}$$

$$\text{air acoustic impedance } Z2 = 4.08 \times 10^{\wedge}2 \text{ [kg/m}^{\wedge}2/\text{s]}$$

$$\text{silicone rubber acoustic impedance } Z3 = 1.0 \times 10^{\wedge}6 \text{ [kg/m}^{\wedge}2/\text{s]}.$$

Substituting the impedance of water and air into the above formula for transmittance, the transmittance becomes almost zero.

In other words, between water and air, and between silicone rubber and air, the transmission of sound waves is almost zero and total reflection occurs.

The acoustic impedance of polystyrene, which is often used as a culture substrate, is Z4=2.4×10^6 [kg/m^2/s]. Therefore, in terms of physical matching between the vibrating body and the culture substrate in the acoustic transmission medium of the present disclosure, it is preferable to use a material having an acoustic impedance close to the above value as the first and second acoustic transmission media.

With respect to the mechanical matching of the vibrating body and the culture substrate, it is preferable that the vibrating body adheres to the culture substrate via the solid. In particular, in the present disclosure, since the vibration waveform of the vibrating body is formed on the cell culture surface of the culture substrate, it is important to have the mechanical matching so that the vibrating body can adhere to the culture substrate over a wide area.

When a commercially available dish type container is used as the culture substrate, if water or a low-molecular material such as glycerin, which easily deforms under low loads, is used as the acoustic transmission medium, when the culture substrate is provided on the vibrating body, a projection portion of the culture substrate directly contacts the vibrating body, resulting in multiple vibration transmission paths. This may complicate the generated vibration and make it difficult to control. The cell detachment method of the present disclosure performs active vibration control. In other words, the vibrating body and the culture substrate are brought into close contact with each other by means of a solid to keep the vibration transmission path constant and to facilitate vibration control.

In the case where the vibrating body and the culture substrate are mechanically matched by the acoustic transmission medium of the present disclosure, the solid material is preferably a material having elasticity such as rubber or gel which is a polymer material, and more specifically, silicone resin, urethane resin, diallyl phthalate resin, unsaturated polyester, epoxy resin, phenol resin, urea resin, melanin resin, natural rubber and the like are mentioned. Among these, silicone rubber is preferable because it has a physical property with little attenuation in the frequency band of the driving frequency (vibration frequency), so that transmission efficiency is high, and furthermore, heat generation of the acoustic transmission medium itself can be suppressed.

(First acoustic transmission medium)

The first acoustic transmission medium is made of a solid material having elasticity at room temperature. In this specification, the room temperature is 20°C or more and 30°C or less, preferably 25°C.

Examples of the solid material having elasticity include rubber and gel which are polymer materials. The polymer rubber material includes, for example, a rubber containing at least one kind of resin selected from the group consisting of a silicone resin, a urethane resin, a diallyl phthalate resin, an unsaturated polyester, an epoxy resin, a phenol resin, a urea resin and a melanin resin, and a natural rubber. More specifically, silicone rubber, urethane rubber, butyl rubber, nitrile rubber, butadiene rubber and latex rubber are mentioned.

Examples of polymer gel materials include hydrogel and polymer gel. Examples of the hydrogel include a hydrogel obtained by combining a hydrophilic polymer material such as polyvinyl alcohol or polyacrylic acid with a crosslinking agent corresponding thereto. Examples of the crosslinking agent include borax and layered clay minerals (clays) as inorganic crosslinking agents, and polyfunctional polymer materials such as dendritic polymers as organic crosslinking agents. Examples of the polymer gel include a polymer gel obtained by combining a polymer material such as an acrylic resin or a urethane resin with a multifunctional compound as a crosslinking agent corresponding to the polymer material.

The first acoustic transmission medium can be used, in which the tanδ obtained by dynamic viscoelasticity measurement at room temperature is less than 1.0 when vibration is applied at a frequency of 1 Hz. Further, when the second acoustic transmission medium is solid at room temperature and vibration of frequency 0.1 Hz is applied, and tanδ of the first acoustic transmission medium and the second acoustic transmission medium obtained by dynamic viscoelasticity measurement at room temperature is $\tan\delta_i$ and $\tan\delta_{ii}$, respectively, it is preferable that $\tan\delta_i < \tan\delta_{ii}$ and/or $\tan\delta_i \geq 1$ is satisfied.

The first acoustic transmission medium preferably contains at least one selected from the group consisting of silicone rubber, natural rubber, urethane gel, and gel material, and particularly preferably contains silicone rubber. Silicone rubber has high transmission efficiency due to its physical property with little attenuation in the frequency band of the driving frequency, and furthermore, heat generation of the acoustic transmission medium itself can be suppressed.

In order to facilitate removal, a surface treatment such as a fluorine coating may be applied to the surface of the first acoustic transmission medium facing the culture substrate or the surface facing the vibrating body.

The acoustic transmission medium is preferably transparent to light in the wavelength region of visible light to directly observe the cell detachment situation.

Various additives may be added to the first acoustic transmission medium for the purpose of controlling physical property such as viscosity, affinity with the second acoustic transmission medium, acoustic impedance with the second acoustic transmission medium, storage stability, and antibacterial action. In addition, the affinity with the second acoustic transmission medium refers, for example, to characteristics such as contact angle and wettability between the first acoustic transmission medium and the second acoustic transmission medium.

As for the thickness of the first acoustic transmission medium, if it is too thick, it tends to reflect ultrasonic waves at the interface in ultrasonic vibration transmission, and vibration attenuation cannot be ignored. If it is too thin, the durability of the membrane encapsulating the second acoustic transmission medium is compromised, and the membrane may also tear or puncture, causing the encapsulated material to come out. Therefore, the total thickness of the first acoustic transmission medium is preferably 5 mm or less when the vibrating body is vibrated.

(Second acoustic transmission medium)

The second acoustic transmission medium is liquid at room temperature or has a tan$\delta$ greater than that of the first acoustic transmission medium when vibration of frequency 0.1 Hz is applied to the second acoustic transmission medium at room temperature.

To be liquid at room temperature means to be liquid between 20°C and 30°C. In particular, the intrinsic viscosity [η] at a room temperature of 25°C, the complex viscosity η* obtained by dynamic viscoelasticity measurement at a room temperature of 25°C, or the viscosity η measured by changing the shear rate with a B-type viscometer or rheometer at a room temperature of 25°C may be small.

Further, in the case of the solid at room temperature, the tan$\delta$ of the second acoustic transmission medium when vibration of 0.1 Hz frequency is applied at room temperature is larger than that of the first acoustic transmission medium. Preferably, a solid having a tan$\delta$ of 1 or more obtained by dynamic viscoelastic measurements at room temperature when a frequency of 0.1 Hz vibration is applied. That is, when the second acoustic transmission medium is a solid and vibration at a frequency of 0.1 Hz is applied, tan$\delta$ of the first acoustic transmission medium and the second acoustic transmission medium obtained by dynamic viscoelastic measurement at room temperature are respectively defined as $\tan\delta_i$ and $\tan\delta_{ii}$, and it is preferable that $\tan\delta_i < \tan\delta_{ii}$ is satisfied and/or $\tan\delta_i \geq 1$.

A flexible gel is cited as a solid that can be a second acoustic transmission medium. As the flexible gel, similarly to the gel exemplified as the first acoustic transmission medium, for example, a hydrogel in which a hydrophilic polymer material such as polyvinyl alcohol or polyacrylic acid is combined with a multibranched polymer material such as borax, a layered clay mineral (clay) or a dendritic polymer as a crosslinking component, a polymer gel in which a polymer material such as an acrylic resin or urethane resin is combined with a multifunctional compound as a corresponding crosslinking agent, and the like are cited.

It is preferable to use the value of tan$\delta$ at the temperature and time scale at which the vibrating body and the culture substrate are mechanically matched by the acoustic transmission medium. That is, tan$\delta$ can be calculated by measuring dynamic viscoelasticity G' and G" when vibration of frequency 1 (Hz) or 0.1 (Hz) is applied at room temperature, more preferably 25°C, using a rheometer or the like, and calculating the dynamic viscoelasticity by using the relational expression tan$\delta$ = G"/G'. Here, G represents shear modulus, G' represents storage shear modulus, and G" represents loss shear modulus. However, the above requirements may be satisfied when the tan$\delta$ of the second acoustic transmission medium is measured. In some cases, the tan$\delta$ of the second acoustic transmission medium cannot be measured. The second acoustic transmission medium may be a liquid at room temperature. In this case, tan$\delta$ may not be measurable.

If the second acoustic transmission medium is a liquid, the viscosity may be high enough not to impair acoustic transmission.

The viscosity can be measured by a B-type viscometer or rheometer at room temperature, more preferably 25°C. Furthermore, it is preferable to adopt the value of viscosity at a shear rate of 1.0 (1/s) when using a 25 mm parallel plate and measuring at a gap of 1.0 mm. Depending on the viscosity of the liquid, appropriate torque values may not be obtained under the above conditions, but in such cases, measurement can be made by changing the diameter and gap values of the plate to achieve the same level of shear rate.

Examples of the liquid include low viscosity liquids such as water, physiological saline, ethanol and diluted ethanol, and mixtures thereof, as well as high viscosity liquids such as glycerin, silicone oil and polymer solutions, gel materials and mixtures thereof. A polymer solution in which the gel is dispersed and swollen in a liquid such as water is also preferable, and more specifically, an ultrasonic gel in which viscoelasticity is adjusted by adding additives such as PVA gel or other thickening agents to a liquid such as glycerin is also preferable. The gel material may be classified as solid or liquid. The gel as a solid has a high content of crosslinking component, and the gel as a liquid has a low content of crosslinking component. In the present specification, a substance for which tan$\delta$ can be measured by the measuring method performed by the inventors is defined as a solid, and a substance for which tan$\delta$ can not be measured is defined as a liquid. This is considered to be related to repulsion as an elastic body. In the following examples, the PVA is a flexible gel as a solid and the ultrasonic gel is a gel as a liquid.

In order to prevent the contents from leaking out when the first acoustic transmission medium encapsulating the second acoustic transmission medium is damaged, the second acoustic transmission medium is preferably a solid having a tan$\delta$ of 1.0 or more when vibration with a frequency of 0.1 Hz is applied at room temperature.

The second acoustic transmission medium may contain various additives for the purpose of controlling physical property such as viscosity, affinity or acoustic impedance with the first acoustic transmission medium, surface tension, storage stability, and antibacterial action.

Examples of additives include the following.

As the additives for adjusting the surface tension, sodium dodecyl sulfate, sodium lauryl sulfate and the like can be mentioned as anionic surfactants.

As the cationic surfactants, ethyl trimethylammonium bromide, benzalkonium chloride, dimethylaminopropyl amide stearate and the like can be mentioned.

As the nonionic surfactants, Contaminon (trademark), Contaminon (trademark) US, Pluronic(trademark) F-68, Tween (trademark) 20, Tween (trademark) 80 and the like can be mentioned.

As the amphoteric surfactants, CHAPS, CHAPSO and the like can be mentioned.

As the storage stabilizers, light stabilizers such as hindered amines, benzophenones and benzotriazoles, and antioxidants such as amines, phenols, sulfur compounds and phosphorus compounds can be mentioned.

As the compounds exhibiting antibacterial activity, benzalkonium chloride, sodium azide and antibiotics such as ampicillin, penicillin and streptomycin can be mentioned.

(Encapsulation of acoustic transmission medium)

The acoustic transmission medium used in the cell detachment method of the present disclosure has a configuration in which a second acoustic transmission medium is encapsulated by a first acoustic transmission medium.

Encapsulation is sufficient if the second acoustic transmission medium is present inside the first acoustic transmission medium between the vibrating body and the culture substrate. For example, the second acoustic transmission medium may be sealed inside the first acoustic transmission medium in the form of a bag, or the first acoustic transmission medium in the form of a bag may be provided with a circulation tube so as not to impair vibration transmission, and the second acoustic transmission medium can be supplied and circulated through the tube. In the case where the second acoustic transmission medium can circulate in the first acoustic transmission medium, it is preferable that after the second acoustic transmission medium is encapsulated within the first acoustic transmission medium in the arrangement step described later, the second acoustic transmission medium is not circulated by a valve or the like in the pressing and detachment step.

As the method for encapsulation, for example, the first acoustic transmission medium in the form of a bag is made, and after injecting the second acoustic transmission medium into the bag, the injection port is sealed. As the sealing method, a method of sealing by melting with heat, a method of sealing by pressure-bonding, a method of sealing by reacting with an adhesive or a raw material compound of the first acoustic transmission medium, a method of sealing by physically clamping, and the like can be mentioned.

As another method of encapsulation, there is a method in which a sheet of the first acoustic transmission medium on the upper surface and the lower surface is prepared, and the upper and lower surfaces are tightly sealed while the second acoustic transmission medium is encapsulated. The cell detachment method of the present disclosure can comprise, as an encapsulation step, an encapsulation step of encapsulating the second acoustic transmission medium into the first acoustic transmission medium.

The first acoustic transmission medium can have at least one protrusion that is convex in a direction facing the second acoustic transmission medium. Preferably, the protrusion has a shape that physically contacts the first acoustic transmission medium on the opposite surface when pressed in the pressing step, and may be, for example, cylindrical, hemispherical, conical, or a combination of a cylinder or a cone and a hemisphere. The protrusion is preferably made of the same material as the first acoustic transmission medium.

The position of the protrusion is preferably a position corresponding to the abdomen (a region having a large amplitude value) where the vibration of the ultrasonic vibration pattern to be applied is strong but may comprise a portion where the vibration is not strong or a portion where the vibration is not caused. The number of protrusions may be at least one. On the other hand, for example, in order to respond to a plurality of vibration patterns, a plurality of protrusions may be arranged at positions corresponding to the abdomen of each vibration pattern.

The first acoustic transmission medium may have at least one column connected across the second acoustic transmission medium.

In the acoustic transmission medium of the present disclosure, the surface facing the vibrating body can have a higher Young's modulus than the surface facing the culture substrate. The high Young's modulus is synonymous with the high elastic modulus, and the Young's modulus can be increased by, for example, increasing the thickness of the first acoustic transmission medium or increasing the amount of multibranched polymer components in the manufacturing of the first acoustic transmission medium.

Preferably, the acoustic impedance difference between the first acoustic transmission medium and the second acoustic transmission medium is small. Further, since the acoustic transmission medium preferably does not contain air, when the second acoustic transmission medium is encapsulated within the first acoustic transmission medium, the second acoustic transmission medium is preferably encapsulated so as not to contain air.

The thickness of the acoustic transmission medium of the present disclosure is preferably 10 mm or less from the viewpoint of reducing vibration attenuation and preventing ultrasonic waves from being reflected at the interface, and is preferably 5 mm or less when vibration is generated in the vibrating body.

The aspect ratio (thickness/size of diagonal line) of the acoustic transmission medium is preferably 0.3 or less. When the aspect ratio is set to 0.3 or less, stability during installation is improved.

The surface of the acoustic transmission medium of the present disclosure facing the vibrating body and the surface facing the culture substrate may be applied with a liquid such as water to enhance consistency before use. The liquid is preferably a liquid with low surface tension.

In the acoustic transmission medium of the present disclosure, the surface facing the vibrating body may be entirely or partially bonded to the vibrating body using an adhesive, a tackifier, or the like to enhance consistency. For adhesives and tackifiers, known adhesives, tackifiers, double-sided tapes, etc. can be used.

(Culture substrate)

The cell detachment method of the present disclosure detaches cells attached to the culture surface of the culture substrate from the culture substrate. Cell attachment includes not only the adhesion of adherent cells but also the presence of cells in contact. That is, attachment of cells to the culture substrate includes not only adhesion of adherent cells but also the presence of cells in contact. That is, attachment of cells to the culture substrate includes, but is not limited to, adhesion of cells to the culture substrate via adhesion factors, and includes retention of cells due to weak interaction with the culture substrate, or simple retention of due to external forces such as gravity.

The culture substrate of the present disclosure refers to any substrate that is used for cell culture and comprises a surface to which cells can attach. The culture substrate includes, for example, a container or a portion thereof for cell culture applications. Examples of cell containers include dishes (also called petri dishes), flasks, plates, tubes, and the like. All of these, for example, products from Corning, Thermo Fisher Scientific and AGC Techno Glass are used as general-purpose disposable containers. In particular, a dish is a typical example of the culture substrate of the present disclosure. Culture substrates with removable lids are commonly used. As used herein, the culture substrate may or may not comprise components such as lids. An example of the shape of the dish is shown in the schematic diagram of Fig. 8. Among them, the bottom surface warpage height 300 in particular varies greatly among model numbers of different manufacturers, among lots, and even within lots. Although mechanical alignment of the culture substrate with the acoustic transmission medium requires consideration of variations in the height of the bottom surface warpage, the use of the first acoustic transmission medium and the second acoustic transmission medium of the present disclosure together facilitates mechanical alignment.

As shown in the schematic diagram of Fig. 8, the bottom surface of a commercially available culture substrate has a projection portion on the outer periphery. The main roles of the projection portion may be to prevent damage to the bottom surface of the substrate, to allow containers to be stacked on top of each other, and to prevent the temperature of the workbench from being transmitted directly to the bottom surface of the substrate when provided on the workbench. When the culture substrate and the acoustic transmission medium are mechanically aligned, the alignment may be performed by avoiding the projection portion or may be performed by covering the projection portion.

The material of the substrate may be any material that is chemically stable and capable of cultivating desired cells. Examples include polyethylene, polypropylene, polycarbonate, polystyrene, polyvinyl chloride, nylon, polyurethane, polyurea, polylactic acid, polyglycolic acid, polyvinyl alcohol, polyvinyl acetate, poly (meth) acrylic acid, poly (meth) acrylic acid derivative, polyacrylonitrile, poly (meth) acrylamide, poly (meth) acrylamide derivative, polysulfone, cellulose, cellulose derivative, polysilicone, polymethylpentene, glass, and metal. Among them, polystyrene is preferable.

(Vibrating body)

The vibrating body of the present disclosure can be used without limitation as long as it generates vibration, and examples include a piezoelectric body and a vibration plate bonded together. When the piezoelectric body is circular, the vibration plate is preferably glass, SUS, or quartz. When the vibration plate is made of glass, SUS or quartz, large amplitude can be output at relatively high driving frequency (vibration frequency) in the ultrasonic region without damage as a vibrating body. The vibrating body can include an ultrasonic vibrator.

In the case of an annular shaped piezoelectric body, the outer diameter size of the vibration plate is preferably equal to the outer diameter size of the piezoelectric body.

The thickness of the vibration plate is preferable such that when the piezoelectric body and vibration plate are bonded and perform flexural vibration, a midpoint in the direction of the flexural thickness, that is, a neutral surface where neither tension nor compression occurs during flexural vibration, is on the vibration plate side in order to efficiently utilize the strain of the piezoelectric body for the flexure.

(Piezoelectric body)

Preferably, the piezoelectric body used for the vibrating body of the present disclosure is an annular shaped piezoelectric body, but the shape of the piezoelectric body that deflects the vibrating body may be an annular shape or a disc shape. To directly observe the cell detachment situation, the annular shape which can secure the field of view of the lower part of the culture substrate is preferable.

The piezoelectric body exhibits piezoelectric characteristics by applying polarization treatment, but as shown in Fig. 10, the polarity of polarization may be changed according to the electrode pattern. The polarization treated piezoelectric body can excite the vibration modes of a standing wave mode and a traveling wave mode by controlling the phase of the input AC voltage for each electrode pattern. A standing wave is generated by applying an AC voltage with a phase difference of 180 degrees to a negatively polarized electrode with respect to a positively polarized electrode. On the other hand, as shown in Fig. 10, when the electrodes are divided into phases A and B on the left and right, if an AC voltage with a phase difference of 90 degrees between the A and B phases is applied, a 2-phase driving traveling wave is generated. In Fig. 10, GND refers to ground, which is used to ground the electrodes located on the back surface.

An example of the electrode pattern is formed by printing Ag, but other than Ag, a noble metal such as Au, Pt, Pd, or a base metal such as Cu may be used as the electrode material. The forming method may be a printing method, a plating method, or a sputtering method.

Although PbZrTiO3 (PZT) is used as the material composition of the piezoelectric body used for the vibrating body, it is preferable to use a lead-free piezoelectric material substantially free of Pb in consideration of environmental regulations. The lead-free piezoelectric material includes BaTiO3 (BT), NaNbO3, BiNaTiO3, BiFeO3, and the like as the main components, and may be a combination of each, or may contain a metal element added to the main components. Especially, it was confirmed that the vibration performance of BaTiO3 (BT) based material was equivalent to that of PbZrTiO3 (PZT). As materials other than ceramics, a single crystal material or a polymer-based piezoelectric material may be used.

The thickness of the piezoelectric body of the vibrating body was calculated based on the relationship between the hardness of the piezoelectric body and the hardness of the vibration plate, because it is preferable that the midpoint in the direction of the flexural thickness, that is, the neutral surface where neither tension nor compressed occurs during the flexural vibration, is on the vibration plate side when the bonded and laminated piezoelectric body and the vibration plate perform flexural vibration, to efficiently utilize the strain of the piezoelectric body element for the flexure.

(Step)

The cell detachment method of the present disclosure may comprise the following steps.

Arrangement step:

A step of providing an acoustic transmission medium on a vibrating body, optionally supplying a second acoustic transmission medium to be encapsulated within a first acoustic transmission medium, and then providing a culture substrate, Pressing step:
A step of applying pressure from the culture substrate or the vibrating body side so that the culture substrate and the vibrating body are in contact with the acoustic transmission medium,

Detachment step:

A step of vibrating the vibrating body while the culture substrate and the vibrating body are in contact with the acoustic transmission medium.

Fig. 5 shows one embodiment of the cell detachment method of the present disclosure.

First, as an arrangement step, a first acoustic transmission medium is provided on a vibrating body (step S210).

Then, a second acoustic transmission medium is supplied so as to be encapsulated within the first acoustic transmission medium (step S220).

Further, a culture substrate is provided on the acoustic transmission medium (step S230).

Next, as a pressing step, pressure is applied from the upper surface of the culture substrate with a weight or the like (step S240).

As a detachment step, ultrasonic application conditions are set (step S250).

Next, the ultrasonic application is started (step S260).

Finally, detached cells are collected (step S270).

It should be noted that the supply of the second acoustic transmission medium to be encapsulated within the first acoustic transmission medium, that is, the encapsulation step is not an essential step in the cell detachment method of the present disclosure, and the supply may be performed separately, or an acoustic transmission medium in which the second acoustic transmission medium is previously encapsulated within the first acoustic transmission medium may be used. Further, applying pressure, i.e., pressing step, is also not an essential step in the cell detachment method of the present disclosure.

(Arrangement step)

In the arrangement step, an acoustic transmission medium is provided on a vibrating body, and if necessary, a second acoustic transmission medium is supplied to be encapsulated within a first acoustic transmission medium, and a culture substrate is provided.

If the acoustic transmission medium is designed to supply and circulate a second acoustic transmission medium, this step supplies and circulates the second acoustic transmission medium and closes the valve to stop supply and circulation during subsequent pressing and detachment steps. By supplying and circulating the second acoustic transmission medium, the amount of the second acoustic transmission medium in the acoustic transmission medium can be controlled, the second acoustic transmission medium can be brought into contact with each other appropriately according to the type and shape of the culture substrate, and the temperature of the acoustic transmission medium can also be controlled by circulating the second acoustic transmission medium.

(Pressing step)

The pressing step in the present embodiment only needs to be a step of pressing so that the culture substrate and the vibrating body come close to each other, and the culture substrate may be displaced so as to be closer to the vibrating body, the vibrating body may be displaced so as to be closer to the culture substrate, or both the culture substrate and the vibrating body may be displaced so as to be closer to each other.

In the pressing step, in a state where the culture substrate and the vibrating body are in contact with the acoustic transmission medium, pressure is applied from the culture substrate side or the vibrating body side to mechanically align the culture substrate and the acoustic transmission medium.

Pressing unit for carrying out the pressing step includes, for example, a method of placing a weight on the lid of the culture substrate, or applying a load uniformly from above on the lid of the culture substrate container using a loading method such as a spring or a leaf spring to fix the upper part of the lid of the culture substrate so as not to move from the upper part and applying a load from the bottom to the upper direction together with the vibrating body.

For the pressing unit, weight loading is preferable in consideration of vibration insulation, but other loading methods can be selected within a range that does not interfere with vibration. For example, a constant load spring can be used as the pressing unit.

As the shape of the weight placed on the lid of the culture substrate, for example, it is preferable to have an annular shape so that cells in the container can be easily observed.

The applied load varies depending on the size of the culture substrate and the applied vibration pattern. For example, in the case of a 35 mm dish, it is preferably between 10 g and 300 g, and in the case of a 60 mm dish, it is preferably between 100 g and 600 g. The load may be a weight or the weight of the culture vessel itself.

If the weight is too heavy, it will suppress and inhibit the vibration itself, and in addition, the culture substrate itself will be bent, causing uneven vibration transmission and leading to a decrease in the detachment rate.

(Detachment step)

In the detachment step, the cell is detached by vibrating the vibrating body while the culture substrate or vibrating body is in contact with the acoustic transmission medium. Furthermore, the detachment step can comprise a step of exciting the vibrating body to vibrate in an ultrasonic band.

Vibration of the vibrating body is not started in the arrangement step, and vibration of the vibrating body is started in the detachment step. For example, in the example of the device shown in Fig. 6, supplying voltage at a resonance frequency drives the vibrating body to generate a desired ultrasonic vibration mode. In this case, during the detachment step, a voltage corresponding to the resonance frequency is supplied to the vibrating body to drive it and generate the desired ultrasonic vibration mode. The power source for applying the above voltage is not shown in Fig. 6.

The vibration pattern can be freely selected according to the cell type and the detachment result. In addition, these vibration patterns may be used singly in the detachment step, or they may be input periodically with changes.

It is preferable that the vibration waveform can vibrate the cell culture surface of the culture substrate at a constant amplitude, for example, an amplitude of 1 $\mu$m. Basically, a resonant vibration can be detected when the frequency is swept from low frequency to high frequency, for example, from 20 kHz to 150 kHz in the ultrasonic range, but the vibration detected on the low frequency side has a large amplitude but a small acceleration. On the other hand, the vibration detected on the high frequency side has a small amplitude but a large acceleration. Therefore, the conditions for imparting a force to weaken the adhesion force of cultured cells to the culture substrate largely depend on the adhesion force of the cells and the size and shape of the cells. Further, since the flow generated in the detachment solution varies with amplitude and acceleration, various combinations exist. In addition, simply using large amplitudes and large accelerations may be undesirable because it promotes the occurrence of cavitation, which causes a decrease in the survival rate of the cell.

The vibration waveform may be a standing wave mode formed as a concentric or circumferentially ordered pattern from the center of a disk-shaped vibration plate, or a traveling wave mode forming a radial vibration waveform on a vibration plate by vibrating the piezoelectric body in a radial direction with a phase change. A vibration waveform can be formed by applying a voltage to each vibrating body at a resonance frequency, and the resonance frequency and the shape of the vibration waveform can be calculated by structural calculation using the shape of the vibrating body, hardness, density, and other physical properties.

Further, when the vibrating body is continuously driven, the resonant frequency shifts to the low frequency side due to heat generation of the piezoelectric body itself, so that the amplitude decreases when the frequency is kept fixed and driven. For this reason, sufficient amplitude can be obtained by using a driving method which suppresses temperature rise due to self-heating by providing a pause time after the amplitude is made for a certain time, or a driving method which repeats sweeping from the high frequency side to the low frequency side within a certain frequency range and the resonance frequency is always passed through even if heat is generated.

(Vibration Evaluation)

The vibration waveform of the vibration plate can be measured by a laser Doppler vibrometer (Graphtec AT7200). The measurement method will be described with reference to the cell detachment device shown in Fig. 6. When the cell detachment device is driven at a desired resonance frequency and voltage, and the surface of the vibration plate 11 is scanned two-dimensionally in the XY direction by a laser, the vibration velocity at each point is obtained, and the amplitude in the Z direction can be calculated from the calculation. As a result, the vibration waveform of the vibration plate 11 can be configured three-dimensionally, and the positions of the abdomen with large amplitude of vibration and the nodes without vibration during driving can be dynamically grasped.

Vibration measurement of the cell culture surface of the culture substrate 21 can also be performed in the same configuration. It is preferable to provide the acoustic transmission medium 13 on the vibration plate 11, deposit a metal, e.g., Ag, on the inner surface of the culture substrate 21 by vapor deposition so that the laser reflects off the culture substrate 21, and provide the culture substrate 21 so that there is no air layer between the culture substrate 21 and the acoustic transmission medium 13. In Fig. 6, the first acoustic transmission medium and the second acoustic transmission medium are collectively shown as the acoustic transmission medium 13.

In the case of a standing wave mode in which the resonance intended to drive the culture substrate 21 provided on the acoustic transmission medium 13 by the cell detachment device is concentric in amplitude, the amplitude of the central portion of the culture substrate 21 is swept near the desired resonance frequency, and the entire cell culture surface of the culture substrate 21 is scanned two dimensionally at the frequency at which the amplitude is largest, whereby the vibration waveform generated on the cell culture surface of the culture substrate 21 can be grasped three-dimensionally.

(Cell)

The cell detachment method of the present disclosure can be applied to any kind of cell without limitation. Examples include various cultured cell lines such as CHO cells derived from Chinese hamster ovary, mouse connective tissue L929 cells, mouse skeletal muscle myoblasts (C2C12 cells), normal diploid fibroblasts derived from human fetal lung (TIG-3 cells), cells derived from human fetal kidney (HEK293 cells), A549 cells derived from human alveolar basal epithelial adenocarcinoma, HeLa cells derived from human cervical carcinoma, cell lines derived from insects, etc., various cultured cell lines such as epithelial cells and endothelial cells constituting various tissues and organs in an organism, skeletal muscle cells, smooth muscle cells, and cardiac muscle cells exhibiting contractility, neuronal cells, glial cells, and fibroblasts constituting the nervous system, primary cultured cells such as hepatic parenchymal cells, hepatic non-parenchymal cells, and adipocytes involved in metabolism in an organism, induced pluripotent stem (iPS) cells, embryonic stem (ES) cells, embryonic germ (EG) cells, embryonic carcinoma (EC) cells, various stem cells such as mesenchymal stem cells, hepatic stem cells, pancreatic stem cells, skin stem cells, muscle stem cells, and reproductive stem cells, or progenitor cells of each tissue, as well as cells induced to differentiate from them.

The cell culture conditions can be appropriately selected according to the cells to be cultured.

In general, an appropriate culture medium is added to the culture substrate 21, and cells of about $1.0 \times 10^1$ cells/cm$^2$ to $5.0 \times 10^4$ cells/cm$^2$ are seeded thereon and cultured at 37°C with a $CO_2$ concentration of 5%. At this time, it is preferable to incubate until the cell occupancy area rate in the culture substrate 21 reaches about 70% to 80%, which is a so-called sub-confluent state.

The culture solution (medium) of the cultured cells can be replaced with the cell detachment solution in the cell detachment step.

Generally, the detachment solution contains proteolytic enzymes. Examples of proteolytic enzymes include trypsin, accutase, collagenase, natural protease, chymotrypsin, elastase, papain, pronase, or their recombinants. However, since proteolytic enzymes dissolve cells, adverse effects on cells are feared. On the other hand, in the cell detachment method of the present disclosure, no proteolytic enzyme is used, and even if the proteolytic enzyme is used, the same effect can be obtained with a very small amount of the proteolytic enzyme, which is effective for suppressing adverse effects on cells.

The cell detachment solution is a solution used to detach cells and may have an action to reduce cell attachment.

The pH of the cell detachment solution is preferably in the neutral range. This is because the neutral range is suitable for cell culture, and the survival rate of the cells can be kept stably high. The pH can be adjusted accordingly with hydrochloric acid, sodium hydroxide, etc. Various buffers are also suitably used to keep the pH stable. The detachment solution can also be a culture medium.

The viscosity of the cell detachment solution can be adjusted appropriately by adding polymers and saccharides.

A solution containing a metal ion chelating agent (hereinafter referred to as "chelating agent") is particularly preferable as a cell detachment solution. This is because cell detachment solutions containing chelating agents can effectively reduce cell adhesion.

Examples of the chelating agent include, but are not limited to, ethylenediaminetetraacetic acid (hereinafter sometimes referred to as "EDTA"), ethylenediamine, ethylenediamine tetramethylene phosphonic acid, glycol ether diamine tetraacetic acid, nitrilotriacetic acid, diethylenetriamine pentaacetic acid, iminodiacetic acid, dihydroxyethylglycine, dicarboxymethyl glutamic acid, ethylenediamine disuccinic acid, etidronic acid, citric acid, gluconic acid, phosphonobutane triacetic acid, and the like. Among them, a chelating agent which forms a chelate with divalent cations is preferred, a chelating agent which forms a chelate with $Ca^{2+}$ and $Mg^{2+}$ is particularly preferred, and ethylenediaminetetraacetic acid is most preferred. When ethylenediaminetetraacetic acid is used as the chelating agent, the pH of the cell detachment solution is preferably 7.0 to 8.0. This is because the high pH in the neutral region, which can keep the cell viability high, can enhance the chelating ability of ethylenediaminetetraacetic acid and can increase the reduction efficiency of the cell adhesion. The chelating agent may be used alone or in combination with two or more chelating agents.

The content of the chelating agent is preferably 0.01mM to 5.0mM.

Within this range, the chelating effect can be reliably obtained, and the lowering of activity due to the presence of an excessive chelating agent can also be suppressed.

The cell detachment solution may contain a hydrophilic polymer comprising a polyalkylene glycol structure. Polyethylene glycol is an example of a hydrophilic polymer comprising a polyalkylene glycol structure. The hydrophilic polymer preferably has a peak molecular weight Mp of 800 to 50,000 as determined by gel permeation chromatography, and more preferably 1200 to 20,000. This is because the polymer has little effect on cells and the thickening effect of the polymer can be suppressed.

(Detachment test)

A detachment test can be performed to evaluate the cell detachment method of the present disclosure. The detachment test can be performed, for example, as follows.

The cell is cultured, and an arrangement step is performed on the substrate obtained by replacing the culture liquid (culture medium) with the detachment solution as necessary, and the vibrating body can also be driven while being maintained at a constant temperature, for example, 25°C, in a warmth keeping instrument as necessary. The driving time is driven with a time sufficient to detach the cells. After the drive, the detached cells are collected by a dropper, and the cells which cannot be collected and are still stuck to the culture substrate 21 are collected by pipetting method. The detachment rate used below is the ratio of the number of cells detached by vibration to the total number of cells detached by vibration and pipetting method, expressed as a percentage. The variance was calculated by determining the detachment rate at n = 10, and the standard deviation of the detachment rate was defined as A for less than 5%, B for 5% to less than 7.5%, C for 7.5% to less than 10%, and D for 10% or more.

Residual cells remaining on the bottom surface of the substrate after cell detachment can be observed using light scattered by a backlight.

(Cell detachment device)

The cell detachment device according to the present disclosure is a cell detachment device for detaching cells attached to a culture surface of a culture substrate by transmitting vibrations generated by a vibrating body to the cells, comprising a vibrating body, an acoustic transmission medium, and a culture substrate installation portion in this order, wherein vibrations of the vibrating body are transmitted to the culture substrate via the acoustic transmission medium,

wherein the acoustic transmission medium comprises a first acoustic transmission medium and a second acoustic transmission medium, wherein the first acoustic transmission medium is a solid having elasticity at room temperature, and the second acoustic transmission medium is encapsulated within the first acoustic transmission medium and is liquid at room temperature or has a tan$\delta$ greater than that of the first acoustic transmission medium when vibration of frequency 0.1 Hz is applied to the second acoustic transmission medium at room temperature.

Fig. 6 shows the configuration of the device of the present disclosure.

As shown in Fig. 6, the cell detachment device of the present disclosure comprises a vibrating body 101 and an acoustic transmission medium 13. The culture substrate installation portion is sufficient if the user can recognize that a culture container is to be provided. In the example in Fig. 6, the opening formed by the vibrating body 101, upper cover 15, and cushioning material 16 is suitable for placing a general-purpose dish, so this opening corresponds to the culture substrate installation portion.

The cell detachment device shown in Fig. 6 shows an example in which a piezoelectric body 12 and a vibration plate 11 are provided as a vibrating body 101 which is a vibration generating part. The vibrating body 101 provided with the piezoelectric body 12 and the vibration plate 11 generates bending vibration in which vibration is amplified by a combination of hardness between the piezoelectric body 12 which expands and contracts by itself and the bonded vibration plate 11. In this example, resonance due to a structural factor of vibrating body 101 is utilized, and a large vibration can be obtained at a resonance frequency. The vibrating body may comprise a vibrator such as an ultrasonic vibrator. By applying a voltage to the piezoelectric body 12 using a power source (not shown), the above expansion and contraction is made.

In Fig. 6, an annular vibrator is shown as a typical vibrating body 101, but a Langevin vibrator or a rectangular vibrator capable of obtaining a large amplitude may be used.

To efficiently transmit the vibrations formed on the vibration plate by resonance to the culture substrate 21 on which the cells are cultured, the acoustic transmission medium 13 is interposed between the vibration plate 11 and the culture substrate 21. When the culture substrate 21 is directly provided on the vibration plate 11, the contact portion becomes a point contact, and the energy is consumed as energy other than vibration such as noise and generation of heat, which causes an efficiency deterioration. Basically, it is preferable that the acoustic transmission medium 13 is provided in an area which sufficiently satisfies the area for detaching off the cells attached to the culture substrate 21.

In order to fix the vibrating body 101, the end of the vibrating body 101 is clamped by the upper cover 15, the lower cover 17, the cushioning material 16 and the bolt 18 shown in Fig. 6. However, the clamp pressure at the end is tightened so as not to inhibit vibration generated in the vibrating plate.

An example is shown in which a weight 14 as a pressing unit for a pressing process is provided on the upper part of the culture substrate and on the lower part of the vibrating body. By moving the weight 14 up and down, pressing and depressing can be performed.

By supplying a voltage at a resonance frequency, the vibrating body is driven to generate a desired ultrasonic vibration mode.

The cell detachment device can be driven by using an AC power supply for supplying a voltage at a resonance frequency to drive the vibrating body and applying a voltage that produces the desired amplitude at a frequency corresponding to the vibration mode capable of forming the desired vibration waveform on the cell culture surface of the culture substrate. At this time, when the voltage of the S-phase, which is the sensor phase provided in the vibrating body, is measured by an oscilloscope, the voltage generated by the distortion of the vibrating body itself can be directly monitored, and the vibrating body can be driven while confirming the vibrating state of the vibrating body. In addition, by providing a thermocouple that can measure the temperature during the cell detachment process in a location that is not affected by vibration, the cell detachment device can be driven while the process is monitored. An infrared thermometer may be used for temperature measurement.

Cases of acoustic transmission media used in the cell detachment methods of the present disclosure are shown in Figs. 1A to 1C, 2A to 2C, 3A to 3C, 4A to 4C, and 9A to 9D.

(First case)

The configuration diagram of the first case of an acoustic transmission medium is shown in Figs. 1A to 1C and 9A.

In the first case of an acoustic transmission medium, a second acoustic transmission medium is encapsulated within a first acoustic transmission medium.

Fig. 1A shows a configuration in which the acoustic transmission medium is in contact with a flat bottomed culture substrate and a vibrating body.

Fig. 1B shows a configuration in which the acoustic transmission medium is in contact with a curved bottomed culture substrate and a vibrating body.

Fig. 1C further shows that the acoustic transmission medium follows the shape of the bottom surface of the culture substrate and the like by applying pressure from the culture substrate or vibration plate.

Fig. 9A illustrates a top view and a cross-sectional view of the acoustic transmission medium in the first case.

(Second case)

The configuration diagram of the second case of the acoustic transmission medium is shown in Figs. 2A to 2C and 9B.

In the second case of the acoustic transmission medium, the second acoustic transmission medium is encapsulated within the first acoustic transmission medium, and the first acoustic transmission medium has a protrusion that is convex in a direction facing the second acoustic transmission medium.

Fig. 2A shows a configuration in which an acoustic transmission medium having a protrusion on the upper surface side of the first acoustic transmission medium is in contact with a flat bottomed culture substrate and a vibrating body.

Fig. 2B shows a configuration in which an acoustic transmission medium having a protrusion on the upper surface side of the first acoustic transmission medium is in contact with a curved bottomed culture substrate and a vibrating body.

Fig. 2C further shows that the acoustic transmission medium of the present disclosure follows the shape of the bottom surface of the culture substrate and the like by applying pressure from the culture substrate or vibration plate. As shown in the figure, the protrusion is preferably configured to contact the first acoustic transmission medium facing each other in a pressed state.

Fig. 9B shows a top view and a cross-sectional view of the acoustic transmission medium of the second case. In the figure, 207 indicates a diameter of the protrusion and 208 indicates a height of the protrusion.

(Third case)

The configuration diagram of the third case of the acoustic transmission medium is shown in Figs. 3A to 3C and 9C.

In the third case of the acoustic transmission medium, the second acoustic transmission medium is encapsulated within the first acoustic transmission medium, and the first acoustic transmission medium has a column connected across the second acoustic transmission medium.

Fig. 3A shows a configuration in which an acoustic transmission medium having the first acoustic transmission medium with a column connected across the second acoustic transmission medium is in contact with a flat bottomed culture substrate and a vibrating body.

Fig. 3B shows a configuration in which an acoustic transmission medium having the first acoustic transmission medium with a column connected across the second acoustic transmission medium is in contact with a curved bottomed culture substrate and a vibrating body.

Fig. 3C further shows that the acoustic transmission medium of the present disclosure follows the shape of the bottom surface of the culture substrate and the like by applying pressure from the culture substrate or vibration plate.

Fig. 9C shows a top view and a cross-sectional view of the acoustic transmission medium of the third case. In the figure, 209 shows a diameter of the column.

(Fourth case)

The configuration diagram of the fourth case of the acoustic transmission medium is shown in Figs. 4A to 4C and 9D.

The fourth case of the acoustic transmission medium is provided with a second acoustic transmission medium supply tube 133 for encapsulating the second acoustic transmission medium within the first acoustic transmission medium.

Fig. 4A shows a configuration in which an acoustic transmission medium having a second acoustic transmission medium supply tube 133 is in contact with a flat bottomed culture substrate and a vibrating body.

Fig. 4B shows a configuration in which an acoustic transmission medium having a second acoustic transmission medium supply tube 133 is in contact with a curved bottomed culture substrate and a vibrating body.

Fig. 4C further shows that the acoustic transmission medium of the present disclosure follows the shape of the bottom surface of the culture substrate and the like by applying pressure from the culture substrate or vibration plate. In this state, the valve of the second acoustic transmission medium supply tube 133 is preferably closed.

Fig. 9D illustrates a top view and a cross-sectional view of the acoustic transmission medium of the fourth case.

[Examples]

[Example 1]

The following examples illustrate examples using the configuration of the first case of the acoustic transmission medium shown in the schematic diagram of Fig. 1A. A Nunc (trademark) Φ35 mm dish manufactured by Thermo Fisher Scientific Inc. was used as the culture substrate. The vibration plate of the cell detachment device was made of glass with an outer diameter of 70 mm and a thickness of 4 mm. The material composition of the piezoelectric body was PZT, the outer diameter was 70 mm, the same as that of the vibration plate, the inner diameter was 57 mm, and the thickness was 2 mm in consideration of the fact that the neutral surface was on the vibration plate side. The cell detachment device was assembled as shown in Fig. 6, and on the vibration plate, an acoustic transmission medium having a shape of height (205) = 2.0 mm, upper surface height (204) = 1.0 mm, lower surface height (206) = 1.0 mm, upper surface thickness (201) = 0.2 mm, lower surface thickness (202) = 0.2 mm, diameter (203) = 30 mm and sealed with water as shown in the schematic view of Fig. 9A was used.

The method of manufacturing the acoustic transmission medium is to prepare molds corresponding to the upper and lower surface shapes of the acoustic transmission medium, prepare silicone rubber raw material (product name: KE-1950-10; manufactured by Shin-Etsu Chemical Co., Ltd.), pour it into the molds, and heat cure it to obtain silicone rubber corresponding to the upper and lower surfaces. This is used as the first acoustic transmission medium. Then, both sides of rubber were bonded together with adhesive, and water was injected as a second acoustic transmission medium through the inlet, and the inlet was sealed with adhesive. The extra inlet was removed to obtain the acoustic transmission medium.

The obtained acoustic transmission medium was provided so that its center aligned with the center of the vibration plate, and then the center of a Nunc (trademark) Φ35 mm dish manufactured by Thermo Fisher Scientific Inc. which was a culture substrate on which cells were cultured, was slowly provided on the acoustic transmission medium so that its center aligned with the center of the vibration plate. The schematic diagram showing the configuration at this time is shown in Fig. 1B. Then, a 100 g weight was placed on the lid of the culture substrate and pressed. The schematic diagram showing the configuration of the acoustic transmission medium after pressing is shown in Fig. 1C.

The cells were cultured as follows. CHO (Chinese Hamster Ovary) cells were seeded at a density of 10,000 cells/cm$^2$ in Nunc (trademark) Φ35 mm dishes manufactured by Thermo Fisher Scientific Inc. and cultured at 37°C in a 5% $CO_2$ environment. The culture medium used was Ham's F12 (manufactured by Thermo Fisher Scientific) supplemented with 10% Fetal Bovine Serum (manufactured by Sigma-Aldrich) and 1% penicillin-streptomycin (10,000 U/ml, manufactured by Thermo Fisher Scientific). Cultures were carried out for 48 hours, and cells were observed with a phase contrast microscope to confirm cell adhesion and proliferation. The cell occupancy rate of the dish was about 80%.


(Detachment of cells)

The culture medium in the dish was removed, the cells were washed with PBS (-), and then immersed in PBS (-) detachment solution for 3 minutes.

Thereafter, the culture substrate was provided in a cell detachment device at an environmental temperature of 25°C, and the cell detachment was performed by frequency sweep oscillation (Frequency 22-27 kHz, Frequency sweep period 1 s, Voltage 90 V) for 2 minutes in a standing wave mode at an ambient temperature of 25°C.

After collecting the detached cells, cells that could not be detached by ultrasonic waves were detached from the dish after ultrasonic detachment using a cell scraper. The number of detached cells was measured on a hemocytometer. The total number of detached cells was determined by combining the number of cells detached by ultrasonic waves and the number of cells detached by a cell scraper thereafter. And the ratio of the number of ultrasonically detached cells to the total number of detached cells was defined as the detachment rate and calculated. As for the dispersion, the detachment rate was calculated with n=10, and the standard deviation of the detachment rate was evaluated as A for less than 5%, B for 5% to less than 7.5%, C for 7.5% to less than 10%, and D for 10% or more.

The evaluation results showed that the detachment rate was 95.4% and the dispersion was rated A.


[Example 2]

In Example 2, the conditions were the same as in Example 1 except that the type of the second acoustic transmission medium was changed as follows. That is, in this Example, an ultrasonic gel (Projelly normal type for Ultrasonography; manufactured by JEX) was used as the second acoustic transmission medium.

The evaluation results showed that the detachment rate was 96.1% and the dispersion was rated A.


[Example 3]

In Example 3, the conditions were the same as in Example 1 except that the type of the second acoustic transmission medium was changed as follows. That is, PVA gel was used as the second acoustic transmission medium.

PVA gel was prepared by the following method. Denka Povar (trademark) K-24E (manufactured by Denka Co., Ltd.) was weighed to a mass of 3.7%, pure water was added, and the total volume was adjusted to 80 mL. PVA was completely dissolved by stirring while applying heat in a constant temperature bath to obtain an aqueous solution of PVA. Next, borax (special grade sodium tetraborate (10-hydrate); manufactured by Kishida Chemical) was weighed, pure water was added to make it 1.0% by mass, and the total volume was made to 16 mL. The borax aqueous solution was gradually added dropwise while stirring the PVA aqueous solution, and PVA gel was obtained by stirring until it became uniform.

The evaluation results showed that the detachment rate was 93.0% and the dispersion was rated A.

[Example 4]

In Example 4, the conditions were the same as in Example 1 except that the shape of the first acoustic transmission medium was changed as follows. That is, as the acoustic transmission medium shown in the schematic view of Fig. 9A, an acoustic transmission medium having a height (205) = 2.0 mm, an upper surface height (204) = 1.0 mm, a lower surface height (206) = 1.0 mm, an upper surface thickness (201) = 0.2 mm, a lower surface thickness (202) = 0.4 mm, and a diameter (203) = 30 mm was used.

The evaluation results showed that the detachment rate was 94.6% and the dispersion was rated A.

[Comparative Example 1]

In Comparative Example 1, the same conditions as in Example 1 were examined except that silicone rubber was used as the acoustic transmission medium.

Silicone rubber with a thickness of 2.0 mm and a hardness of 30° was used as an acoustic transmission medium.

The evaluation results showed that the detachment rate was 80.1% and the dispersion was rated D.

(Summary)

Table 1 summarizes the results of the configurations of the above examples and comparative examples.

[Table 1]

Table 1

| | Acoustic transmission medium | | | | | | | | Total thickness mm | Aspect ratio | Detachment ratio % | Dispersion |
| | First acoustic transmission medium | | | | Second acoustic transmission medium | | | | | | | |
| | Material | 1Hz tan $\delta$ | 0.1Hz tan $\delta$ | Total thickness mm | Material | Viscosity mPa·s | 1Hz tan $\delta$ | 0.1Hz tan $\delta$ | | | | |
|---|---|---|---|---|---|---|---|---|---|---|---|---|
| Example 1 | Silicone rubber | 0.13 | 0.12 | 0.4 | Water | 0.9 | Liquid | Liquid | 2 | 0.067 | 95.4 | A |
| Example 2 | Silicone rubber | 0.13 | 0.12 | 0.4 | Ultrasonic gel | 6.6x10^4 | Liquid | Liquid | 2 | 0.067 | 96.1 | A |
| Example 3 | Silicone rubber | 0.13 | 0.12 | 0.4 | PVA gel | 1.1x10^6 | 0.86 | 1.7 | 2 | 0.067 | 93 | A |
| Example 4 | Silicone rubber | 0.13 | 0.12 | 0.6 | Water | 0.9 | Liquid | Liquid | 2 | 0.067 | 94.6 | A |
| Comparative Example 1 | Silicone rubber | 0.13 | 0.12 | 2 | - | - | - | - | 2 | 0.067 | 80.1 | D |

The present disclosure is not limited to the embodiments described above, and various changes and modifications may be made without departing from the spirit and scope of the present disclosure. Accordingly, the following claims are appended hereto in order to make the scope of the present disclosure public.

The present application claims priority based on Japanese Patent Application No. 2023-118584 filed on July 20, 2023, the description of which is incorporated herein by reference in its entirety.

[Reference Signs List]

11 vibration plate
12 piezoelectric body
13 acoustic transmission medium
131 first acoustic transmission medium
132 second acoustic transmission medium
14 weight
15 upper cover
16 cushioning material
17 lower cover
18 bolt
21 culture substrate
22 culture substrate upper cover
101 vibrating body

**Claims**

1. A cell detachment method for detaching cells attached to a culture surface of a culture substrate from the culture surface,

   wherein the cell detachment method detaches the cells from the culture surface by vibrating a vibrating body in a state where an acoustic transmission medium is provided between the culture substrate and the vibrating body, wherein the acoustic transmission medium comprises a first acoustic transmission medium and a second acoustic transmission medium,
   wherein the first acoustic transmission medium is a solid having elasticity at room temperature,
   and the second acoustic transmission medium is encapsulated within the first acoustic transmission medium and is liquid at room temperature or has a $\tan\delta$ greater than that of the first acoustic transmission medium when vibration of frequency 0.1 Hz is applied to the second acoustic transmission medium at room temperature.

2. The cell detachment method according to claim 1, wherein the first acoustic transmission medium has at least one protrusion that is convex in a direction facing the second acoustic transmission medium.

3. The cell detachment method according to claim 1, wherein the first acoustic transmission medium has at least one column connected across the second acoustic transmission medium.

4. The cell detachment method according to any one of claims 1 to 3, wherein the first acoustic transmission medium comprises at least one selected from the group consisting of a silicone rubber, a natural rubber, a urethane gel, and a gel material.

5. The cell detachment method according to any one of claims 1 to 4, wherein the second acoustic transmission medium is a solid having a $\tan\delta$ of 1.0 or more when vibration with a frequency of 0.1 Hz is applied at room temperature.

6. The cell detachment method according to any one of claims 1 to 5, wherein the second acoustic transmission medium is a liquid at room temperature.

7. The cell detachment method according to any one of claims 1 to 6, wherein the second acoustic transmission medium comprises at least one selected from the group consisting of a liquid, a polymer solution, and a gel material.

8. The cell detachment method according to any one of claims 1 to 7, wherein the first acoustic transmission medium comprises a silicone rubber and the second acoustic transmission medium comprises water.

9. The cell detachment method according to any one of claims 1 to 8, wherein a surface of the acoustic transmission medium facing the vibrating body has a higher Young's modulus than a surface of the acoustic transmission medium facing the culture substrate.

10. The cell detachment method according to any one of claims 1 to 9, further comprising an encapsulation step wherein the second acoustic transmission medium is encapsulated within the first acoustic transmission medium.

11. The cell detachment method according to any one of claims 1 to 10, wherein the vibrating body generates vibrations in an ultrasonic band.

12. The cell detachment method according to any one of claims 1 to 11, wherein the cell detachment method comprises a pressing step of pressing the culture substrate and the vibrating body to bring them close together.

13. A cell detachment device for detaching cells attached to a culture surface of a culture substrate by transmitting vibrations generated by a vibrating body to the cells, comprising

a vibrating body,
an acoustic transmission medium, and
a culture substrate installation portion in this order,
and configured so that vibrations of the vibrating body are transmitted to the culture substrate via the acoustic transmission medium,
wherein the acoustic transmission medium comprises a first acoustic transmission medium and a second acoustic transmission medium,
wherein the first acoustic transmission medium is a solid having elasticity at room temperature,
and the second acoustic transmission medium is encapsulated within the first acoustic transmission medium and is liquid at room temperature or has a $\tan\delta$ greater than that of the first acoustic transmission medium when vibration of frequency 0.1 Hz is applied to the second acoustic transmission medium at room temperature.

14. The cell detachment device according to claim 13, wherein the vibrating body comprises an ultrasonic vibrator.

15. The cell detachment device according to claim 13 or 14, further comprising a pressing unit for pressing the culture substrate and the vibrating body to bring them close together.

# FIG. 1A

21
131
132
101

# FIG. 1B

21
131
132
101

# FIG. 1C

PRESSURE

21
131
132
101

# FIG. 2A

# FIG. 2B

# FIG. 2C

PRESSURE

# FIG. 3A

# FIG. 3B

# FIG. 3C

PRESSURE

# FIG. 4A

# FIG. 4B

# FIG. 4C

PRESSURE

# FIG. 5

START

PROVIDE AN ACOUSTIC TRANSMISSION MEDIUM
ON A VIBRATING BODY — S210

SUPPLY A SECOND ACOUSTIC TRANSMISSION MEDIUM
SO AS TO BE ENCAPSULATED WITHIN
THE FIRST ACOUSTIC TRANSMISSION MEDIUM AS NEEDED — S220

PROVIDE A CULTURE SUBSTRATE — S230

PRESS THE CULTURE SUBSTRATE — S240

SET ULTRASONIC CONDITIONS — S250

START THE ULTRASONIC APPLICATION — S260

COLLECT DETACHED CELLS — S270

END

# FIG. 6

# FIG. 7A

## FIG. 7B

## FIG. 7C

## FIG. 7D

FIG. 8

300

# FIG. 9A

# FIG. 9B

# FIG. 9C

# FIG. 9D

# FIG. 10

# EP 4 741 495 A1

## INTERNATIONAL SEARCH REPORT

| | International application No. |
|---|---|
| | **PCT/JP2024/025676** |

### A. CLASSIFICATION OF SUBJECT MATTER

***C12N 5/071***(2010.01)i; ***C12M 3/00***(2006.01)i
FI: C12N5/071; C12M3/00 Z

According to International Patent Classification (IPC) or to both national classification and IPC

### B. FIELDS SEARCHED

Minimum documentation searched (classification system followed by classification symbols)

C12N5/071; C12M3/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | WO 2019/044804 A1 (KEIO UNIVERSITY) 07 March 2019 (2019-03-07) | 1-15 |
| A | JP 2019-030260 A (KEIO UNIVERSITY) 28 February 2019 (2019-02-28) | 1-15 |
| A | JP 2018-042534 A (KEIO UNIVERSITY) 22 March 2018 (2018-03-22) | 1-15 |
| A | WO 2016/047368 A1 (SCREEN HOLDINGS CO., LTD.) 31 March 2016 (2016-03-31) | 1-15 |
| A | WO 2023/079905 A1 (HITACHI, LTD.) 11 May 2023 (2023-05-11) | 1-15 |
| A | JP 2008-092857 A (OLYMPUS CORPORATION) 24 April 2008 (2008-04-24) | 1-15 |
| A | KURASHINA, Yuta et al., Enzyme-free release of adhered cells from standard culture dishes using intermittent ultrasonic traveling waves, Communications Biology, 2019, vol. 2, Article number: 393, pp. 1-12 | 1-15 |

☐ Further documents are listed in the continuation of Box C.   ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **03 September 2024** | **01 October 2024** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

**INTERNATIONAL SEARCH REPORT**
Information on patent family members

International application No.

**PCT/JP2024/025676**

| Patent document cited in search report | | | Publication date (day/month/year) | Patent family member(s) | Publication date (day/month/year) |
|---|---|---|---|---|---|
| WO | 2019/044804 | A1 | 07 March 2019 | (Family: none) | |
| JP | 2019-030260 | A | 28 February 2019 | (Family: none) | |
| JP | 2018-042534 | A | 22 March 2018 | (Family: none) | |
| WO | 2016/047368 | A1 | 31 March 2016 | (Family: none) | |
| WO | 2023/079905 | A1 | 11 May 2023 | JP 2023-69062 A | |
| JP | 2008-092857 | A | 24 April 2008 | (Family: none) | |

Form PCT/ISA/210 (patent family annex) (July 2022)

**REFERENCES CITED IN THE DESCRIPTION**

**Patent documents cited in the description**

- JP 2018042534 A
- JP 6325363 B
- JP 2023118584 A